# EUROPEAN PATENT APPLICATION

(11) **EP 1 293 769 A2**
(43) Date of publication of application: **19.03.2003**
(21) Application number: 02020008.5
(22) Date of filing: 05.09.2002
(51) Int. Cl.: G01N 25/32, G01N 27/16

(54) **Flammable gas sensor and gas concentration measurement method and apparatus**

(30) Priority: 07.09.2001 JP 2001271238; 25.06.2002 JP 2002184291; 05.07.2002 JP 2002198038
(71) Applicant: National Institute of Advanced Industrial Science and Technology, Tokyo 100-8921 (JP)
(72) Inventor: Shin, Woosuck, c/o AIST Chubu, Nagoya-shi, Aichi 463-8560 (JP); Murayama, Norimitsu, c/o AIST Chubu, Nagoya-shi, Aichi 463-8560 (JP)
(74) Representative: Ebner von Eschenbach, Jennifer

(57) **Abstract**

The present invention relates to a flammable gas detection sensor that is a gas detection sensor which converts heat generated by the catalytic reaction between the catalyst component and a flammable gas to a voltage signal by a thermoelectric conversion effect and detects this signal as a detection signal, and is characterized by comprising as constituent elements thereof a catalyst component for initiating a catalytic reaction when in contact with a gas to be detected, and a film of thermoelectric conversion material which converts local temperature differences brought about by heat generated by the reaction to voltage signal; and also relates to a flammable gas concentration measurement method and a measurement apparatus thereof.

## Description

### TECHNICAL FIELD

The present invention relates to a gas detection sensor for a flammable gas, and more particularly to a novel type of gas detection sensor in which both roomtemperature operation and high response can be ensured, integration in a film configuration can be achieved, and gas can be detected stably and reliably by adopting a system in which local temperature difference variations brought about by heat generated by a catalytic reaction of a catalyst component with flammable gas are converted to a voltage signal, and this signal is detected as a detection signal.

The present invention also relates to a method and apparatus for measuring the concentration of fuel gas, and more particularly to a high-concentration fuel gas concentration measurement method and apparatus whereby a high-concentration fuel gas can be measured with high accuracy by a process in which part of the fuel gas is sampled and diluted with an air stream, and in which the diluted gas is measured with the aid of a gas sensor or other element whereby local temperature difference variations brought about by heat generated, for example, by a catalytic reaction of a catalyst component with flammable gas are detected as voltage signals.

### BACKGROUND ART

The importance of sensors for hydrogen and other flammable gases is expected to increase with increased popularity of fuel cells. Although high-sensitivity sensors for flammable gases are currently being developed and commercially implemented, issues related to ensuring lower operating temperatures, improved safety, and the like will still need to be resolved in order to allow sensors having low operating temperature, high selectivity, and other functions to be used on a wider scale in general applications. It is therefore imperative that proposals be made concerning new technological elements from the standpoint of materials development.

In conventional practice, sensors for detecting flammable gas are broadly divided into semiconductor-type variable-resistance sensors and integrated-circuit sensors. Most of the flammable gas sensors available on the market as gas sensor elements are semiconductor-type variable-resistance sensors in which tin oxide, which is a semiconductor oxide, is used as a sensor material, and gas is detected by registering resistance variations in the sensor materials due to the surface adsorption of gas molecules or the catalytic combustion on the surfaces of gas molecules. The recently reported sensors of this kind are configured such that a bridge circuit is assembled from a sensor element and a temperature compensation element composed of an inert material, an appropriate voltage is impressed in advance on the sensor element to heat the element to a high temperature, and the potential difference generated during contact with gas is read from the bridge circuit.

The integrated circuit types developed so far are based on an operating principle whereby, theoretically, charge variations induced by gas molecules on the surface of a sensor element are utilized, but because any defects of the element material are electrically charged, it is pointed out that the sensors have drawbacks in terms of reliability, and no practical applications have so far been realized. Integrated-circuit sensors featuring light/surface wave/pyroeleotrio effect and the like have recently been developed, but numerous technical issues have yet to be resolved before these sensors can find practical applications.

The aforementioned variable-resistance sensors cannot perform properly unless they are heated to several hundred degrees Celsius, making it imperative that new sensors capable of efficiently operating at low temperatures be developed. This type of sensor is still plagued by technological issues such as low response speed and the considerable recovery time (about several tens of minutes) needed to return the system to the original state. These sensors must be inspected before use after a power interruption, are subject to annual inspections in order to examine the effect of drifting or the like, and have other features that make these sensors difficult to operate and maintain.

Several problems are encountered when a sensor operates at a high temperature.
1) The first is related to safety. In the case of currently available commercial sensors for hydrogencontaining flammable gases, the element temperature must be kept at 300°C or greater, and the operating temperature rises even more during detection of flammable gases, creating a possibility the sensors will become ignition sources. For this reason, the element must be covered with a metal wire mesh or other measures must be taken, creating problems in terms of safety.
2) Considerable electric power is consumed in order to maintain high element temperature. A particular consequence of this is that it becomes difficult to secure a power supply for portable applications.
3) Because it is kept at a high temperature, the element is difficult to integrate on a silicon chip. With the exception of gas sensor elements, the elements on the silicon chip have low heat resistance, and the gas sensor element and the silicon chip must be thermally insulated from each other. Although it is technologically possible to provide thermal insulation using porous films, the element already has a complex structure because part of the chip is kept at a high temperature.

New sensor principles devoid of the drawbacks of conventional gas sensor elements have already been reported in technical journals (*Sensors and Actuators,* 8 (1985), p. 251). This particular type of sensor is designed for detecting hydrogen gas, and electrodes are disposed at the two ends of a tin oxide ceramic bar in a configuration in which a platinum coating film is formed on one side, and a silver coating film is formed on the other side.

The sensor operates such that when heat is generated by a catalytic reaction between hydrogen gas and a platinum coating film, the temperature rises on one side of the tin oxide bar, the temperature difference between the two ends creates a thermoelectromotive force in the tin oxide due to the Seebeck effect, and the force represents a voltage signal for the hydrogen gas. Since this type of sensor can adequately operate at room temperature, there is no need to heat the element and no concern that ignition will occur.

A feature of such sensors, however, is that tin oxide as an electromotive force generator is a material that responds with great sensitivity to hydrogencontaining flammable gases, so the voltage signal readings are not necessarily limited to the weak heat signals from the catalyst. Tin oxide is a typical material for variable-resistance sensor elements that has sensitive reaction to hydrogen and the like at temperatures ranging from near room temperature to 400°C and undergoes exceptionally large resistance variations (Popular Edition: Sensor Technology, 1998, Fuji Technosystem Corp., ISBN 4-938555-64-6). For this reason, reliability-related problems are encountered when low heat from a catalyst needs to be accurately detected. Thermoelectromotive force is expected to decrease with lower resistance, so these materials need to be replaced by materials that are unaffected by detected gas and are capable of generating high thermoelectromotive force.

The sensor elements reported herein belong to a bulk type shaped as a bar, and this shape limits the scope of their application. Three examples can be cited.
1) Considerable heat capacity, low response speed
2) Extreme difficulty in forming a sensor element on a silicon chip (semiconductor chip)
3) Considerable element dimensions, complicated operations involved in fabricating series-circuit structures and other elements used to obtain stronger voltage signals

The importance of sensors for hydrogen and other flammable gases is expected to increase even further in the near future with increased popularity of fuel cells. Among these sensors, detection sensors for preventing explosions of hydrogen gas are expected to be in particular demand in the future, but there are as yet very few examples in which sensors have been developed for detecting high-concentration hydrogen (4% to 97%, which are the explosive limits of hydrogen). Semiconductor variable-resistance sensors and Pd-alloy resistance sensors are conventionally known as such hydrogen sensors.

The hydrogen sensors currently available on the market are semiconductor variable-resistance sensors, which are sensors in which gas is detected by registering resistance variations in the sensor material due to the surface adsorption of gas molecules or the catalytic combustion on the surfaces of gas molecules. This principle is disadvantageous in that the signal becomes saturated at a hydrogen concentration of 0.2% or greater. However, hydrogen sensors need to be able to detect concentrations of up to several percent to be practical. For example, a Japanese gas sensor manufacturer has reported that their commercial sensors are capable of detecting concentrations of up to 0.2%, and the development of sensors capable of detecting up to 2% has also been reported recently (December 2001). These new sensors are still disadvantageous, however, in that the relation between gas concentration and signal level has low linearity, and the signal represents an exponential output.

Pd-alloy resistor sensors have been put on the market by DCH (DCH Technology, Inc., USA), a manufacturer of hydrogen-related products who has commercialized hydrogen sensors for high-concentration gases. A Pd-Ni alloy is used as an element material for the Robust Hydrogen Sensor (TM) of the same company, but this material is of a type that readily stores hydrogen and allows gas to be detected by registering resistance variations and other changes that affect the sensor material in the process, making it possible to sense hydrogen concentrations ranging from 0.5% to 100%. Such elements have inadequate durability, however. In addition, the following two technological difficulties are encountered because the detection principle is based on resistance variations: first, Pd alloys, which are the actual sensor members, have small resistance variations, and peripheral circuits are therefore required to convert these variations into signals, and, second, the resistance of these sensor members is dependent on temperature, so a mechanism must be provided to keep the element temperature constant.

The inventors have previously developed sensors for detecting voltage signals obtained by converting the local temperature differences resulting from the generation of heat by a catalytic reaction between flammable gas and a catalyst component (Japanese Patent Application 2001-271238). These sensors are characterized by having a highly linear concentration measuring capacity with respect to highly concentrated fuel gases whose concentration ranges from 1% to 10%. These sensors have a wide measurement range but cannot measure concentrations of up to 100%, and hence cannot be regarded as high-concentration sensors capable of directly measuring concentrations of up to 100% in the same way as the semiconductor variable-resistance sensors described above.

The semiconductor variable-resistance sensors and Pd-alloy variable-resistance sensors described above, as well as all other sensors whose operating principle is based on the resistance variations of detection members, are characterized by the fact that the resistance variations induced by variations in the element temperature act as noise in relation to the detection signal, necessitating measures to minimize the noise-producing variations in the element temperature. For example, a Pd-alloy variable-resistance gas sensor contains two types of peripheral circuits for controlling element temperature. Control boards are separately mounted for other sensor elements. Various advantages can be achieved by directly incorporating temperature control circuits into elements, but this approach increases the number of parts, complicates the manufacturing steps, and increases development and production unit costs. When, for example, control boards are provided to an area outside an element, limitations are imposed on the thickness and length of electric wires in elements in which resistance variations serve as sources of signals, and adjustments need to be made at each place of installation.

Even elements with such temperature control mechanisms have low reliability when the detected gas is part of a high-temperature atmosphere or when the measurement medium is close to 0°C. so the service temperature often ranges between 0°C and 40°C. A variety of temperatures can be encountered in the case of measured fuel gases used in actual applications, so a wider range of service temperatures is required. For example, the chassis temperature may rise to 100°C or greater in applications involving fuel-cell vehicles, so the drawbacks of existing elements prevent these elements from being used in such applications, and developing novel sensor elements and gas detection methods capable of generating highly reproducible output signals that are independent of the ambient temperature is considered to be an important technical issue in this technological field.

As a result of extensive research conducted in view of the prior art and aimed at overcoming the shortcomings of flammable gas sensors operating on the thermoelectric conversion principle, the inventors perfected the present sensor in which 1) high sensitivity can be maintained, 2) integration with a silicon chip is made possible, and 3) fast response can be achieved by the use of a thermoelectric conversion material having a film shape.

It is an object of the present invention to provide a flammable gas detection sensor in which operation at room temperature, fast response, and high selectivity can be ensured.

As a result of extensive research conducted in view of the prior art and aimed at developing a new flammable gas measurement method designed to accurately measure high-concentration flammable gases while radically overcoming the shortcomings of the prior art, the inventors perfected the present invention upon discovering that the desired objectives can be achieved by combining a process in which part of the fuel gas is sampled and diluted with an air stream, and a process in which the diluted gas is measured using a specific flammable gas detection sensor.

Specifically, it is an object of the present invention to provide a new flammable gas concentration measurement method capable of accurately measuring a high-concentration flammable gas.

It is another object of the present invention to provide a flammable gas concentration measurement apparatus to be employed in the above-mentioned measurement method.

### DISCLOSURE OF THE INVENTION

The present invention provides a flammable gas sensor, a method for accurately measuring flammable gas concentration, and a measurement apparatus thereof.

The present invention relates to a flammable gas detection sensor in which heat generated by a catalytic reaction of a catalyst component with flammable gas is converted to a voltage signal by a thermoelectric conversion effect, and this signal is detected as a detection signal, wherein the gas detection sensor is characterized by comprising as constituent elements thereof a catalyst component for initiating a catalytic reaction when in contact with the gas to be detected, and a film of thermoelectric conversion material whereby local temperature differences brought about by heat generated by the reaction are converted to a voltage signal; also relates to a flammable gas concentration measurement method for measuring the concentration of flammable gas in a fuel gas line, wherein the method is characterized by comprising as constituent elements thereof (1) sampling part of the flammable gas, (2) diluting the sample with an air stream at a specific dilution ratio, (3) measuring the diluted gas by a gas sensor, and (4) measuring the concentration of the flammable gas on the basis of the measured value; and further relates to a measurement apparatus thereof.

The result achieved by the present invention is that a gas detection sensor can be obtained in which operation at room temperature and integration with a silicon chip is made possible, resistance variation induced by the detected gas is minimal, high reliability can be achieved, specific gases can be selectively identified and quantitatively measured, and excellent reliability, fast response (in terms of seconds), and cyclic characteristics can be ensured.

Next, the present invention will be described in further detail.

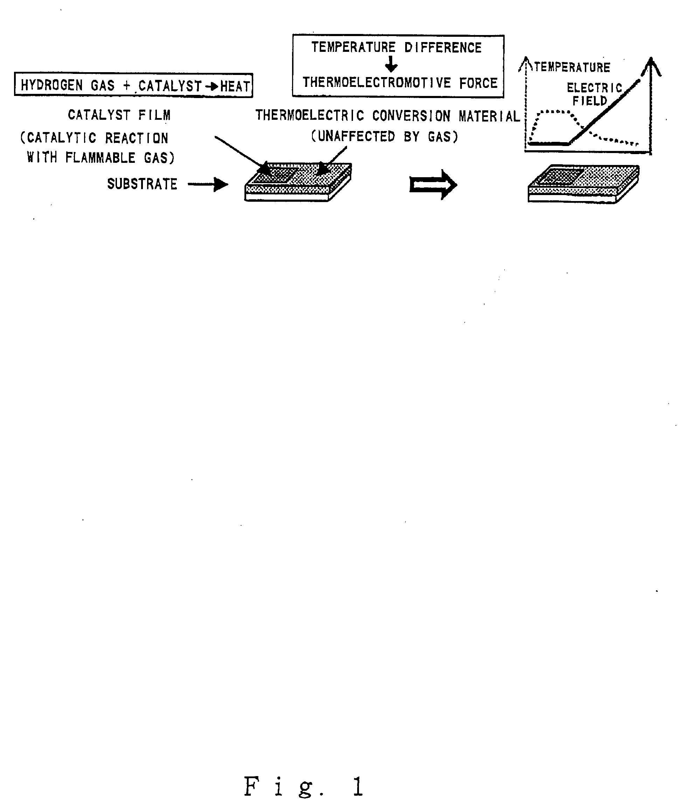

The first embodiment of the present invention provides a flammable gas detection sensor configured as shown in Fig. 1 and aimed at overcoming the shortcomings of a conventional sensor for detecting flammable gas in a catalytic reaction. The sensor can be fashioned into any structure with an appropriate combination of a catalyst component and a film of thermoelectric conversion material formed in contact therewith. Specifically, a film of SiGe (which is a film of thermoelectric conversion material for performing thermoelectric conversion functions) is formed on an alumina substrate, and a film of platinum (which is a catalyst component for performing catalytic functions) is then formed on the right half of the surface thereof, yielding an element. Electrodes are affixed to both the left and right edges of the film in order to gauge voltage. These electrodes preferably consist of silver paste, but are not limited thereto. Nor are the positioning or method of attachment of the electrodes limited.

Platinum (Pt), palladium (Pd), and gold (Au) are used as the above-mentioned catalyst components. Hydrogen, carbon monoxide, methane, and propane can be identified, and mixtures of these gases can be identified and quantitatively measured using these elements individually or in combinations.

This is because the gold catalyst reacts only with carbon monoxide, the platinum catalyst reacts only with hydrogen at room temperature, and methane and propane exhibit reactions with different metals at high temperatures of 100°C or greater (*Chemistry and Chemical Industry,* 24, pp. 4-11, 1973, by Tatsuo Yamanaka).

Next, it is vital to use a thermoelectric conversion material that has high thermoelectric conversion capabilities and has low reactivity with gas. Preferred examples of such materials include, but are not limited to, BiTe compounds, PbTe compounds, FeSi compounds, SiGe compounds, skutterdite compounds, half-Heusler intermetallic compounds, metal oxide compounds, and the like, and any material having superior thermoelectric conversion capabilities can be similarly used. In this case, the term "has low reactivity with gas" refers to materials with minimal gas-induced variations in resistance and thermoelectromotive force.

More specifically, NiO compounds doped with alkali metals can be LixNi₁₋ₓO or NayLixN_{1-y-x}O (*x*: approximately 0.024, *y*: approximately 0.1, *z*: approximately 0.01) (Japanese Patent No. 3051922), and are thermoelectric conversion materials with a simple process. SiGe compounds have the composition Si_{0.8}Ge_{0.2} and are excellent thermoelectric conversion materials with a long history of technological development, and have particularly been used in thermoelectric generators for space exploration because of their excellent thermoelectric characteristics and high mechanical strength in high-temperature areas.

The above-mentioned catalyst component serving as a catalyst may, for example, be fashioned by vapor deposition into a film with a thickness of several nanometers on part of the thermoelectric conversion material. An alumina substrate with a thickness of 0.5 mm and a purity of 95% can be cited as an example of such a substrate, but no particular restrictions are imposed in this aase. A thick plate can be used in the absence of a substrate. As a way of suppressing the thermal conduction of the substrate, it is also possible to adopt a structure in which a film is formed on the substrate, and the substrate is then removed by etching.

The present invention is characterized in that the slightly elevated temperature from the catalytic reaction of the flammable gas is converted to a voltage signal (sufficiently large to be easily gauged) by optimizing the thermoelectric conversion principle of the thermoelectric conversion material, thereby allowing the system to operate adequately at room temperature.

As a result of various experiments conducted by the inventors, it has been concluded that thermoelectric conversion material is the most suitable for the components of a sensor element. The following three reasons can be cited. Specifically, 1) thermoelectric conversion materials have substantially constant thermoelectromotive force and generates signals in proportion to the weak heat from the catalyst due to minimal resistance variations induced by the flammable gas; 2) thermoelectric conversion materials can have enhanced sensitivity because of their ability to generate a greater thermoelectromotive force than that of a common electroconductive material at the same resistance; and 3) thermoelectric conversion materials can prevent in-plane thermal conduction, can provide more accurate detection, and are capable of operating with enhanced sensitivity due to the comparatively low thermal conductivity thereof.

Because of a film configuration, the gas detection sensor of the present invention has the following merits: 1) possesses small heat capacity and fast reaction characteristics; 2) can be integrated with a silicon chip; and 3) can readily generate voltage without amplifier cirouits as a result of a simple variation in the printing pattern.

The gas detection sensor of the present invention is extremely effective in detecting flammable gas as a result of the fact that the thermoelectric conversion characteristics of the film of thermoelectric conversion material used to detect weak heat from the catalyst on the sensor surface are not affected by the detected gas, making it possible to obtain a flammable gas detection sensor with a linear relationship between the detection output and the flammable gas concentration.

The gas detection sensor of the present invention can be used to identify hydrogen, carbon monoxide, methane, and propane in a simple and efficient manner, and is extremely effective in identifying and quantitatively measuring these mixed gases as a result of the fact that varying the type of catalyst material on the sensor surface and combining single or different types of elements provides selectivity for the detected gas.

The second embodiment of the present invention is a flammable gas concentration measurement method characterized in that part of flammable gas is sampled and diluted with an air stream, and the concentration of the flammable gas is measured using a gas sensor. In the present invention, a thermoelectric conversion gas sensor (referred to hereinbelow as a "thermoelectric gas sensor") whose operating principle is based, for example, on thermoelectric conversion of heat generated from the catalyst, is used as the above-mentioned gas sensor. This gas sensor is capable of measuring gas with high concentrations of 10% or greater, and can therefore be made to respond to concentrations of up to 100% in a procedure in which the sensor is combined with a gas mixer, the measured gas is diluted with air, and the diluted gas is measured. However, the present invention is not limited to the above-mentioned gas sensor, and any appropriate gas sensor such as a variable-resistance semiconductor sensor can be employed. The present invention provides, as a means to achieve these objectives, a flammable gas concentration measurement apparatus characterized by comprising as constituent elements thereof a sampling unit for sampling part of the flammable gas, a mixer unit for diluting the sample with an air stream, and a gas sensor unit for measuring the diluted gas.

The present invention also provides a method for measuring the concentration of flammable gas, preferably with the aid of a gas detection sensor whose gas sensor unit may, for example, be configured such that heat generated by a catalytic reaction between the flammable gas and a catalyst is converted to a voltage signal by a thermoelectric conversion effect, and this signal is detected as a detection signal, and also provides the aforementioned apparatus in a configuration featuring a flammable gas detection sensor whose constituent elements comprise a catalyst component for initiating a catalytic reaction when in contact with the detected gas, and a film of thermoelectric conversion material whereby local temperature differences brought about by the heat generated by the reaction are converted to a voltage signal.

In the present invention, fuel gas is diluted with air, but increasing the extent of dilution when diluting one part of the fuel gas with air is disadvantageous in that it becomes difficult to perform mechanical processing and actual flow control in the measurement method and apparatus. To measure concentrations of up to 100% with a gas sensor involved in actual detection, it is necessary to use a device capable of measuring concentrations of up to 10% (which is 1/10 of the actual concentration) if a technically simple dilution ratio of 1/10 is adopted. In preferred practice, the present invention may, for example, use a thermoelectric gas sensor as the gas sensor capable of detecting such high concentrations.

Next, the diluting mixer will be described. Currently commercially available diluting systems can be divided into two categories, one of which involves mixing and diluting the sample gas and the diluted gas at a constant ratio using an orifice by controlling the flow induced by constant pressure, and the other of which involves continuous variation at a dilution ratio of approximately 1/20-1/100 with a glass capillary and mass flow controller. Combinations of these two systems are also possible. The apparatuses of either of these systems are commercially valued from ¥800,000 to ¥2,500,000. It is believed that less expensive equipment can also be produced and distributed because a dilution ratio of 1/20 or less is technically simple, but the actual circumstances are such that developmental examples of so-called gas sampling (in which measured gas is diluted with air) are few due to a lack of demand for dilution ratios of about 1/10.

Examples of devices or mechanisms with dilution ratios of about 1/10 in the present invention include, but are not limited to, apparatuses comprising a pressure regulator and a simple valve capable of regulating the flow rate; simple apparatuses in which a flow regulating valve is fixed as an orifice when the dilution ratio is set; and apparatuses having functions for introducing the gas to be measured (fuel gas) through an orifice and mixing the gas with a constant air flow. In the present invention, the term "air stream" refers to a stream containing air and an equivalent or comparable gas. In the present invention, a gas measuring sensor capable of responding to concentrations of up to 100% can be readily produced by employing a thermoelectric gas sensor with a wide range of measurement concentrations, and combining this element with a simple diluting mixer.

Next, a thermoelectric gas sensor preferably used in the present invention will be described. A platinum (Pt) catalyst may, for example, be preferably used for the above-mentioned thermoelectric gas sensor. In this case, high catalyst activity is exhibited with respect to hydrogen gas at temperatures that start at room temperature, and, as shown in the embodiments hereinbelow, a catalytic reaction of nearly 100% occurs at temperatures of 80°C or greater, and the heat generated from the catalyst has no temperature dependence. Specific heat of the material constituting the element is substantially constant at room temperature, and the temperature gradient induced by catalytic heating is substantially constant regardless of temperature. This means that a gas sensor output independent of temperature can be obtained if the temperature of the element is 80°C or greater.

In the present invention, the above-mentioned thermoelectric gas sensor can be appropriately used as an element devoid of temperature dependence. Since the principle of this thermoelectric gas sensor is that temperature differences alone are the source of signals, a gas sensor devoid of temperature dependency is possible if the Seebeck coefficient does not depend on temperature. A material such as nickel oxide has a large Seebeck coefficient at low temperatures, the value of which decreases with an increase in temperature while conductivity increases. However, at approximately 100°C the variations in the Seebeck coefficient decrease, and then increase again at 200°C or greater. Consequently, the temperature dependence of the Seebeck coefficient is extremely small in this range.

A thermoelectric gas sensor with little temperature dependence in this temperature range can be obtained with an element design involving the use of such thermoelectric material. A detailed report on the temperature dependence of the Seebeck coefficient of nickel oxide is made by the inventors in two technical papers: "Li-Doped Nickel Oxide as a Thermoelectric Material," by W. Shin, N. Murayama, *Jpn. J. Appl. Phys. 2,* vol. 38, No. 11B L1336-8 (1999)," and "High performance p-type thermoelectric oxide based on NiO," by W. Shin, N. Murayama, *Materials Letters,* vol. 45, p. 302 (2000)." Furthermore, at this temperature range, the Seebeck coefficient seldom changes, and it is possible to reduce the noise brought about by the resistance of the element itself by improving conductivity.

The present invention employs a method in which a part of flammable gas is sampled and diluted with an air stream and the concentration of the flammable gas is measured using a gas sensor, and also employs a flammable gas concentration measurement apparatus comprising as constituent elements thereof a sampling unit for sampling part of the flammable gas, a mixer unit for diluting the sample with an air stream, and a gas sensor unit for measuring the diluted gas. The specific structure of this method and apparatus can be properly designed according to the intended purpose thereof. These structures can, for example, be arbitrarily designed by applying microfabrication techniques. Specifically, in the present invention, *i*-TAS (micro total analysis system) and MicroTAS, which are believed to have originated from the synergistic effects of MEMS (micro-electro-mechanical systems) techniques and sensor techniques, can be appropriately used in the present invention. In the present invention, such expansion allows highly efficient miniature apparatuses and elements to be constructed and makes it possible to obtain a broader range of applications (MEMS and *i*-TAS; Fundamentals of Microfabrication, 2002, CRC Press, ISBN 0-8493-0826-7).

The present invention relates to a method for measuring flammable gas concentration and a measurement apparatus thereof, and consists of a method comprising (1) sampling part of the flammable gas, (2) diluting the sample with an air stream at a specific dilution ratio, (3) measuring the diluted gas by a gas sensor, and (4) measuring the concentration of the flammable gas on the basis of the measured value; and a flammable gas concentration measurement apparatus characterized by comprising as constituent elements thereof a sampling unit for sampling part of the flammable gas, a mixer unit for diluting the sample with an air stream, and a gas sensor unit for measuring the diluted gas. Through the use of the above-mentioned flammable gas concentration measurement method and measurement apparatus thereof, the present invention makes it possible to measure the concentration of high-concentration flammable gas within the range of optimum detection capabilities of the gas sensor.

Consequently, numerous measurement methods can be set up by properly selecting the type, function, and other attributes of the gas sensor in accordance with the intended use. Specifically, using, for example, a gas sensor in the form of a variable-resistance semiconductor sensor with a measurement range of 10 ppm to 0.2% makes it possible to measure gas concentrations from 0.01 to 2% if the dilution ratio is 1/10. In another example, using a gas sensor in the form of a thermoelectric conversion gas sensor whereby local temperature difference variations brought about by heat generated by a catalytic reaction between a catalyst component and a flammable gas are detected as electrical signals makes it possible to measure flammable gas concentrations with fast response within a concentration range of 0.25% to 100% and at widely varying temperatures (from 80°C to 180°C) if the dilution ratio is 1/10. Furthermore, if the gas sensor is a combination of a variable-resistance semiconductor system and a thermoelectric system, measurements can be made within a concentration range of 0.01 to 100%.

### BRIEF DESCRIPTION OF THE DRAWINGS

Fig. 1 depicts an example of a sensor structure;
Fig. 2 depicts the response characteristics of a voltage signal from a sensor reacting with hydrogen gas;
Fig. 3 depicts the relationship between the voltage signal and temperature of the detected gas;
Fig. 4 depicts an example of a sensor structure;
Fig. 5 depicts a schematic diagram of a method and apparatus for measuring the concentration of high-concentration flammable gas;
Fig. 6 depicts the relationship between hydrogen gas concentration and the voltage signal obtained using a high-concentration hydrogen gas sensor;
Fig. 7 depicts the response characteristics of a high-concentration hydrogen gas sensor operating at 80°C;
Fig. 8 depicts the relationship between the element operating temperature of a thermoelectric gas sensor and the voltage signal corresponding to different hydrogen concentrations; and
Fig. 9 depicts the relationship between the element operating temperature of a thermoelectric gas sensor and the voltage signal in a high-concentration hydrogen gas sensor.

### DESCRIPTION OF SYMBOLS

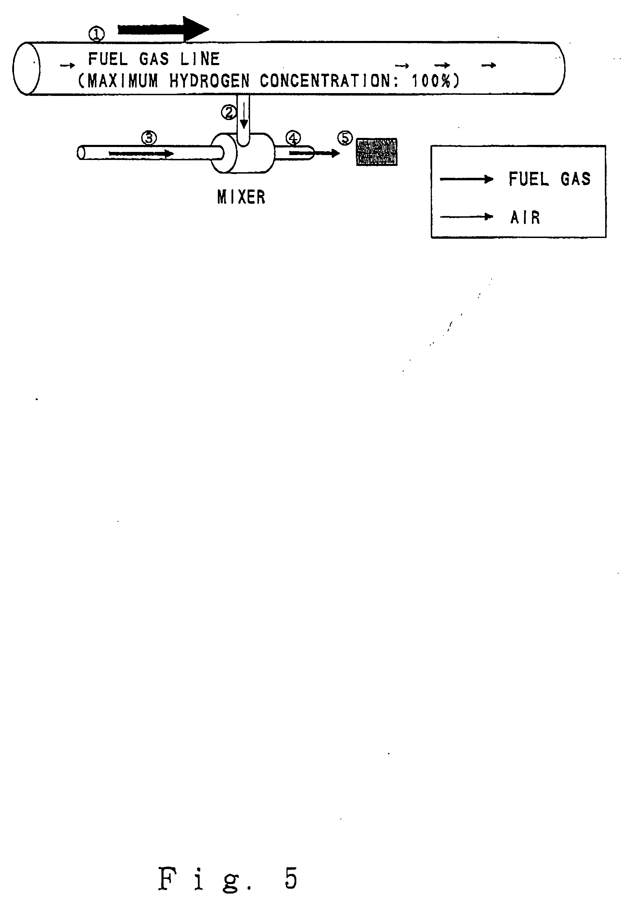

### (Description of Symbols in Fig. 5)

(1) fuel gas line containing high-concentration hydrogen
(2) removal of part of mixed gas from bypass tube
(3) air stream for diluting high-concentration fuel gas to be measured
(4) hydrogen-mixed gas diluted by air
(5) thermoelectric conversion gas sensor

### BEST MODE FOR CARRYING OUT THE INVENTION

Next, the present invention will be described in detail based on embodiments, but the present invention is by no means limited by these embodiments.

### Embodiment 1

Sodium carbonate and lithium oxide were admixed into nickel oxide, calcined for 6 hours at 850°C, and crushed to obtain a powder. The powder was mixed with a vehicle made of terpineol and ethyl cellulose, formed into a paste, printed onto an alumina substrate with a thickness of 0.5 mm and a purity of 95%, and fired for 2 hours at 1100°C to produce a thick film of thermoelectric conversion oxides. Platinum, which served as a catalyst, was subsequently formed in a thickness of several to several tens of nanometers on half of the film surface with the aid of a sputtering device, producing a gas detection sensor.

### Embodiment 2

Phosphorus was admixed into an SiGe alloy (Si 80%, Ge 20%) in a ratio of 0.3 at%, and the mixture was crushed in a planetary ball mill to an average grain diameter of several micrometers or less. The interior of the container was replaced with Ar gas during crushing to prevent oxidation, and silicon nitride was used for the container and the balls to prevent contamination. The mixture was fired using an electric firing apparatus for two hours at a maximum pressure of approximately 30 MPa and a maximum temperature of 1100°C, producing a fired compact. A thin film of SiGe was produced with the aid of a sputtering apparatus by employing the fired compact as a sputtering target. Platinum, which served as a catalyst, was subsequently formed in a thickness of several to several tens of nanometers on half of the film surface with the aid of a sputtering apparatus, producing a gas detection sensor.

### Embodiment 3

In this embodiment, the gas response characteristics of the gas detection sensors fabricated in embodiments 1 and 2 were analyzed. The catalyst area was 0.4 cm², and the mixed gas flow rate was 100 mL/min. The conditions indicated constituted a range in which the results were substantially proportionate to the catalyst surface area and the mixture flow rate up to a multiplication factor of about 3. In other words, under conditions identical to those indicated here, the temperature differences and voltage signal induced by heat increase by a factor of about 2 if the catalyst area is doubled.

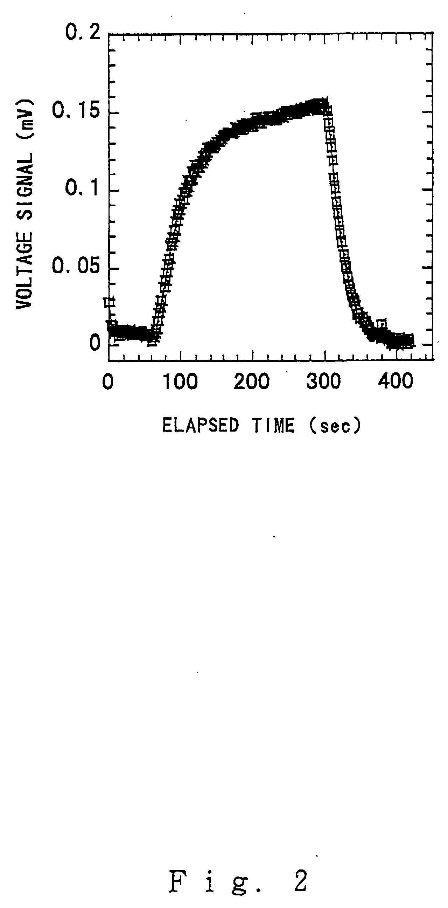

Fig. 2 depicts the response characteristics of a voltage signal from the gas detection sensor of embodiment 1 reacting with hydrogen gas. The temperature of the element during measurement was approximately 29°C, and mixed air containing about 1% hydrogen was used as the detected gas. The mixed gas started to flow in 60 seconds, and air was caused to flow in 300 seconds. The generation of a voltage signal was confirmed during the flow of the mixed gas. The resistance variation of the gas detection sensor during measurement was 1% or less and did not depend on the presence or absence of the mixed gas.

Tables 1 and 2 depict the response characteristics of the gas detection sensors. Response speed is denoted by T90 as the rise time between the moment the mixed gas starts to flow and the moment the voltage reaches 90% of the maximum value, and TD90 as the time between the moment the air starts to flow again and the moment the voltage starts to decrease and reaches 90% of the maximum voltage. The fact that the gas detection sensor of the present invention has a film configuration is believed to be the reason that the response speed can be gauged in increments of about 50 seconds. Another noteworthy result is that excellent cyclic characteristics can be obtained because TD90 is short and the original signal level can be rapidly restored.

The temperature dependence of the response characteristics of the detection sensor varied greatly around an operating temperature of 70°C. Because the calorific value increased in proportion to temperature from room temperature to 70°C, the voltage signal also increased in substantially linear proportion. The calorific value and voltage signal variations decreased above at 70°C or greater and were entirely independent of temperature. Table 1 shows the characteristics thereof at the representative temperature of 100°C.

As shown in Tables 1 and 2, the hydrogen concentration dependence of the response characteristics of the detection sensor showed substantially linearly proportionate results when varying between 1%, 3%, and 10%. In other words, quantitative measurement can be easily conducted with the flammable gas detection sensor of the present invention because of a linear relationship between the detected output and the flammable gas concentration.

**Table 1**

| Response characteristics of detection sensor of embodiment 1 for hydrogen/air mixed gas | | | | | |
|---|---|---|---|---|---|
| Operating temperature, °C | Hydrogen gas concentration, % | Temperature difference generated, °C | Voltage signal, mV | T90, sec | TD90, sec |
| 29 | 1 | 0.34 | 0.15 | 100 | 45 |
| 29 | 3 | 1.2 | 0.40 | 58 | 48 |
| 29 | 10 | 1.9 | 0.61 | 55 | 55 |
| 100 | 1 | 2.1 | 0.92 | 40 | 40 |
| 100 | 3 | 3.9 | 1.8 | 45 | 42 |
| 100 | 10 | 9.0 | 4.2 | 55 | 43 |

**Table 2**

| Response characteristics of detection sensor of embodiment 2 for hydrogen/air mixed gas | | | | | |
|---|---|---|---|---|---|
| Operating temperature, °C | Hydrogen gas concentration, % | Temperature difference generated, °C | Voltage signal, mV | T90, sec | TD90, sec |
| 29 | 1 | 0.11 | 0.012 | 60 | 45 |
| 29 | 3 | 1.0 | 0.89 | 56 | 49 |
| 29 | 10 | 1.8 | 0.21 | 55 | 54 |

### Embodiment 4

The detection sensor obtained using platinum as a catalyst and depicted in the above-mentioned embodiments has substantially no response at room temperature to other types of flammable gas. Except for hydrogen, methyl alcohol and carbon monoxide are reported to cause a catalytic reaction with platinum at comparatively low temperatures (*Chemistry and Chemical Industry*, 24, pp. 4-11, 1973, by Ryoyu Yamanka). Response characteristics towards methyl alcohol and carbon monoxide were examined, but no heat generation or voltage signal could be measured for either compound at 100°C or less.

It is apparent from the above that a detection sensor that uses platinum as a catalyst in the embodiments introduced here responds only to hydrogen gas at operating temperatures of 100°C or less and has excellent hydrogen gas selectivity.

### Embodiment 5

Although a silicon single crystal has low thermoelectric conversion capabilities, an experiment was oonducted in which a gas detection sensor having the same structure and area as in embodiment 1 was fabricated (except that a silicon single crystal was used as a thermoelectric conversion component), and response characteristics with hydrogen gas were compared in order to obtain a thermoelectric conversion effect. The calorific value was substantially identical to that of the device in embodiment 1, but the resulting temperature difference was about 0.2°C at room temperature and 0.8°C at 100°C (that is, less than about 1/5 compared with embodiment 1) under conditions in which the hydrogen gas concentration was 3%, and the voltage signal decreased accordingly. It was confirmed that the reason the temperature difference was lower was because of the high heat conductivity of the silicon single crystal (150 W/mK, a value greater than 4 W/mK, which was the measured value of a NiO compound doped with alkali metals and used as the thermoelectric conversion material in embodiment 1), and also that it is effective to use thermoelectric conversion material with comparatively low heat conductivity in the form of a thermoelectric conversion film.

### Embodiment 6

Pulverized Li- and Na-doped nickel oxide was mixed with a vehicle (made of terpineol and ethyl cellulose), formed into a paste, printed onto an alumina substrate, dried for 5 minutes at 200°C, and fired for 2 hours at 1000°C, producing a thick film of thermoelectric conversion oxides. A thin film of platinum, which served as a catalyst, was subsequently formed in a thickness of several to several tens of nanometers on half of the film surface by rf sputtering, producing a gas detection sensor.

The areas on the film surface of the resulting pt/NiO thick-film hydrogen sensor were as follows: Al₂O₃/2.0 × 1.0 cm² for the substrate, pt/0.9 × 0.5 cm² for the catalyst, and NiO/1.8 × 0.5 cm² for the thermoelectric conversion oxide. The heat characteristics of the above-mentioned gas detection sensor were examined using H₂, CO, CH₄, i-C₄H₁₀, CH₃OH, and C₂H₅OH (concentration 3%) as the detected gases at element temperatures of 35, 45, 65, 85, 100, end 125°C.

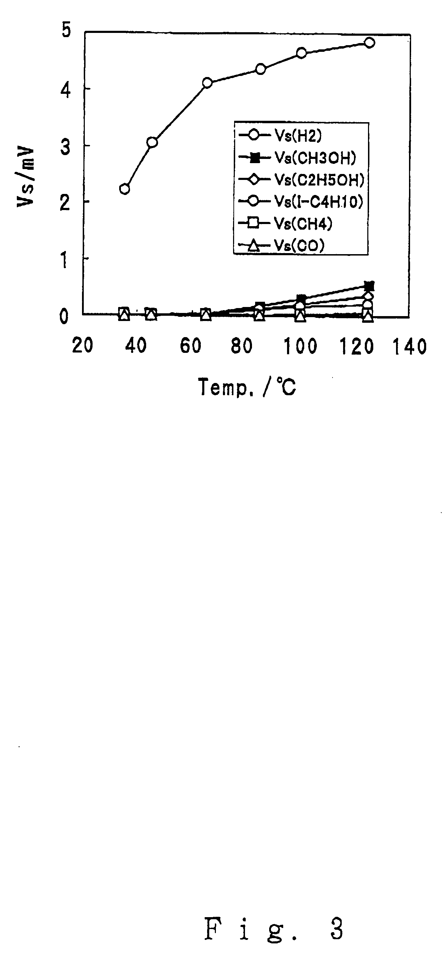

Table 3 depicts the detected voltage signal Vs and the selectivity of hydrogen gas in relation to other flammable gases. Fig. 3 depicts the relationship between voltage signal and temperature for the detected gas.

**Table 3**

| T/°C | H₂ | CO | | CH₄ | | i-C₄H₁₀ | | C₂H₅OH | | CH₃OH | |
|---|---|---|---|---|---|---|---|---|---|---|---|
| | Vs | Vs | S | Vs | S | Vs | S | Vs | S | Vs | S |
| 125 | 4.849 | 0.025 | 194 | 0.05 | 97 | 0.218 | 22 | 0.370 | 13 | 0.559 | 9 |
| 100 | 4.647 | 0.025 | 186 | 0.048 | 97 | 0.176 | 26 | 0.216 | 22 | 0.307 | 15 |
| 85 | 4.355 | 0.025 | 174 | 0.028 | 156 | 0.124 | 35 | 0.128 | 34 | 0.175 | 25 |
| 65 | 4.118 | 0.02 | 206 | 0.018 | 229 | 0.01 | 412 | 0.03 | 137 | 0.039 | 106 |
| 45 | 3.048 | 0.01 | 305 | 0.013 | 234 | 0.01 | 305 | 0.01 | 305 | 0.01 | 305 |
| 35 | 2.219 | 0.005 | n | 0.01 | 222 | 0.005 | n | 0.005 | n | 0.005 | n |

The above results show that the aforementioned gas detection sensor has high selectivity towards hydrogen gas and that although some activity towards methanol and ethanol is observed at high temperatures of 85°C or greater, selectivity towards hydrogen is exceptionally high at low temperatures.

### Embodiment 7

In this embodiment, flammable gas concentration was measured using a flammable gas concentration measuring apparatus containing the thermoelectric gas sensor developed by the inventors.

### 1. Fabrication of Gas Detection Sensor

A thermoelectric gas sensor was fabricated by a procedure in which a thermoelectric film was formed by a printing method, the film was fired, and a platinum catalyst film was formed.

### (1) Paste Preparation and Printing

Sodium carbonate and lithium oxide were admixed into nickel oxide, calcined for 6 hours at 850°C, and crushed to obtain a powder. A paste was subsequently prepared from the resulting oxide powder. A container was first placed on a balance, the powder was introduced in an amount of 2 g, and a builder (2 g) was then added in drops. The product was introduced and mixed in a revolving kneader, the mixing was continued for 2 minutes, the process was terminated 1 minute after bubble out, and the paste preparation process was completed.

The paste was applied to an alumina substrate with the aid of a printer. The mesh used had the following specifications: 0.5 × 1.8 cm² for the openings, stainless steel 230 for the screen, 220 × 260 mm² for the frame, and 45° for the spreading method. After the paste had been applied, the coated substrate was placed on a hot plate with a temperature of 200°C and dried for 5 minutes. The process was repeated several times, yielding a thick film having the required thickness. The number of paint cycles and the film thickness were related in the following manner: film thicknesses (in µm) of 13.3, 25.6, 35.5, and 56.0 were obtained when the number of cycles was 1, 2, 4, and 6, respectively. Specifically, a thickness of about 10 µm could be obtained with a single paint cycle under these conditions.

### (2) Firing of Thermoelectric Film

The dried thick film was kept for 20 minutes at 500°C to vaporize the organic matter. Firing was then performed for 2 hours. The fired film generated a strong voltage signal when the firing temperature was set to 1100°C. This was due to a lower carrier concentration and a higher Seebeck coefficient. However, this also brought about high resistance and readier noise generation. In addition, the 1100°C film was prone to breakage and had inadequate adhesion, indicating that 1000°C is more appropriate for the firing temperature.

### (3) Vapor Deposition of Platinum Catalyst Film

Platinum catalyst was vapor-deposited in a thickness of several nanometers on half the surface of a thick film, yielding a sensor. Although thin-film catalysts are expected to deliver sufficiently high catalyst activity at a film thickness of several nanometers, experiments conducted by the inventors indicated that higher catalyst activity can be obtained by making the Pt film thicker. Two types of Pt films were used to obtain such film thicknesses with the aid of an RF sputtering apparatus.

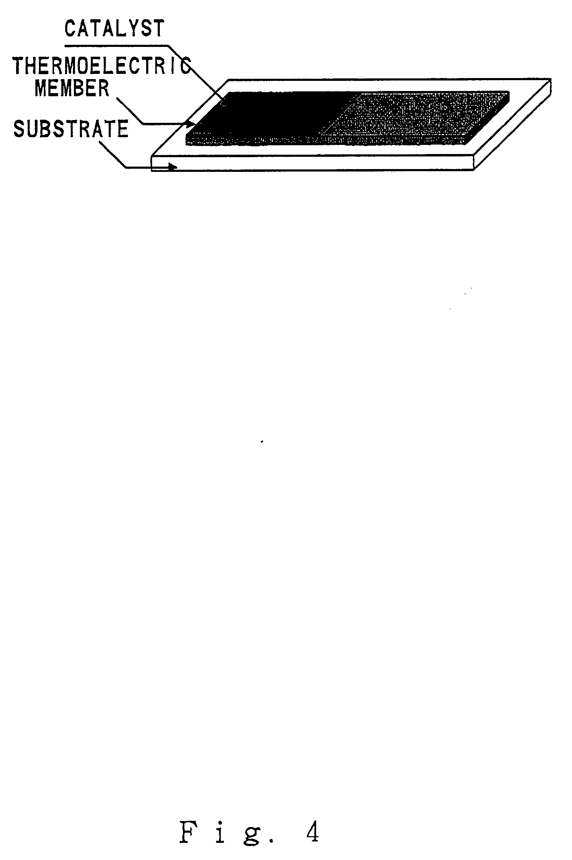

The sputtering conditions were selected such that the vapor deposition pressure was set to about 4 × 10⁻² torr and the sputtering output to 1.0 kV and 50 mA. It was possible to confirm by direct observation that a film pressure of about 50 nm was obtained when sputtering and vapor deposition were continued for 12 minutes under these conditions. In the present experiments, 1-minute sputtering (corresponds to 4 nm) and 3-minute sputtering (corresponds to 12 nm) were performed. Fig. 4 is a schematic drawing of the thermoelectric gas sensor.

### 2. Measurement of High-concentration Flammable Gas Concentration

Concentration of a high-concentration flammable gas was measured by combining a thermoelectric gas sensor and a mixing component.

### (1) Apparatus Structure and Measurement Method

A flammable gas measurement apparatus comprising as constituent elements thereof a sampling unit for sampling part of flammable gas from a flammable gas line, a mixer unit (mixing component) for diluting the sample with air, and a gas sensor unit (thermoelectric gas sensor) for measuring the diluted gas was fabricated, and the concentration of the high-concentration flammable gas was measured. Fig. 5 is a schematic diagram of the method for measuring the concentration of a high-concentration flammable gas. As shown in this drawing, a small amount of fuel gas 1 (high-concentration hydrogen mixed gas based on nitrogen) containing a high concentration of hydrogen at a flow rate of 1.0 L/min was diverted through a bypass 2 and sampled into a mixing component. At this time, the flow rate of the diverted gas was 40 mL/min. Air (high-purity air) 3 was subsequently fed to the mixing component at a rate of 360 mL/min, and the fuel gas was diluted to 1/10. The 1/10-diluted fuel gas 4 having a flow rate of 400 mL/min was finally measured with a thermoelectric hydrogen sensor 5, and the flammable gas concentration was measured.

The apparatus (diluting mixer) for admixing air in a specific dilution ratio (1/10) in accordance with the above-described method was configured using a pressure controller (regulator) and a flowmeter fitted with a needle valve. The pressure on the primary side of the pressure controller was set to 5 atm, which is the pressure commonly used with fuel gases and air. The fuel gas used was obtained by mixing 100% hydrogen gas and 100% nitrogen gas with the aid of a similar diluting mixer. Each flow rate was adjusted with a flowmeter fitted with a needle valve in the same manner, creating a fuel gas flow with a constantly varying hydrogen concentration. Since such a method produces large errors when low fuel gas concentrations are involved, the experimental reliability was maintained by adopting two-step dilution.

### (2) Measurement Results

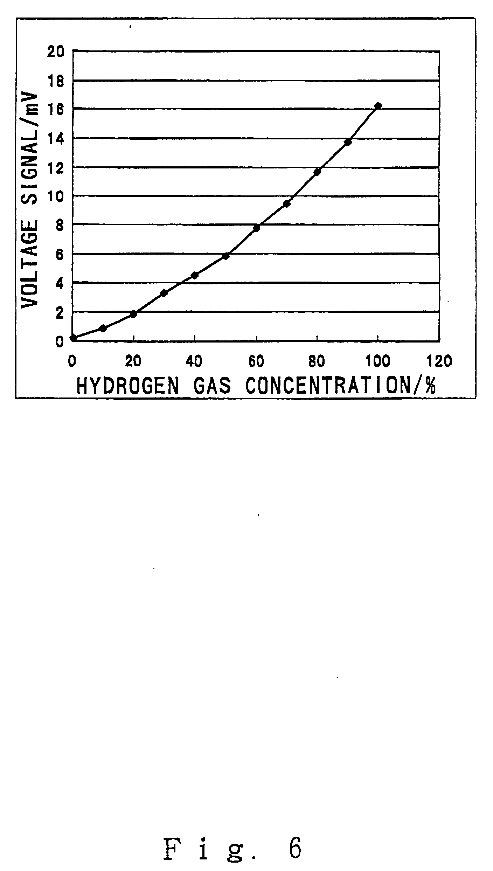

The above-described thermoelectric gas sensor was used to measure voltage signals observed when high-concentration hydrogen gas was diluted with air and the concentration thereof was varied. Fig. 6 depicts the relationship between the concentration of the high-concentration hydrogen gas at a gas sensor element temperature of 80°C and the voltage signals detected by the thermoelectric hydrogen sensor. It can be seen in the drawing that the hydrogen concentration and voltage are in a substantially linear relation in a concentration range of 0.25% to 100%. Because the dilution ratio in this case is 1/10, the gas with 100% concentration is diluted to 1/10 of this value (10%), brought into contact with the gas sensor, and caused to output a voltage signal.

In particular, this element exhibited an excellent linear relationship of signal level versus gas concentration and had an error range of about 10% even when the following simplified formula was used: Hydrogen concentration (%) = 7 × Signal voltage (mV). The existence of this type of excellent linearity of signal level versus gas concentration means that flammable gases can be measured across a concentration range of 0.25% to 100% by the measurement method of the present invention.

### (3) Evaluation of Response Characteristics

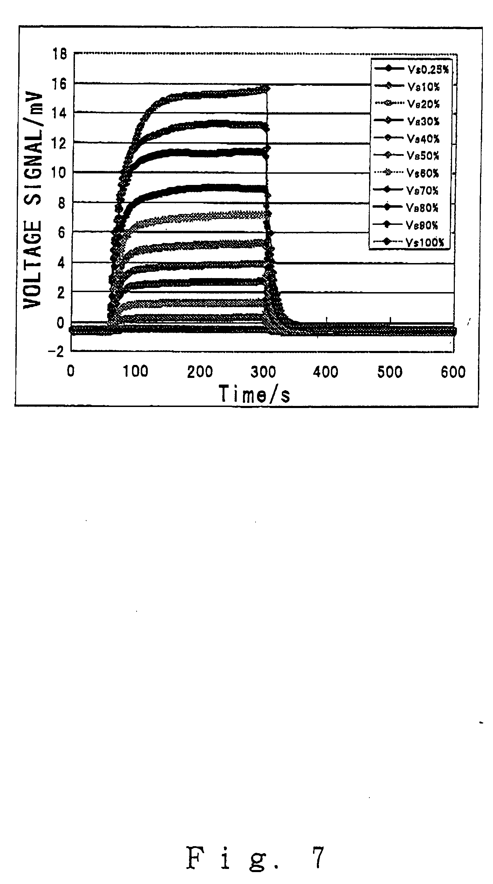

The response characteristics of the inventive measurement apparatus were evaluated. The results are shown in Fig. 7. As shown in Fig. 4, the response characteristics during operation at 80°C have a rise period of several seconds in the concentration range of 0.25% to 100% and that the time (T90) needed to reach the 90% level is 1 minute or less, indicating that the apparatus performs with sufficiently fast response as a leakage sensing element. Another feature of the measurement apparatus is that very short time is needed to regain the zero level, which allows the measurement apparatus to respond to concentration variations at any time and makes the apparatus useful from the standpoint of practical application. The time (TD90) needed to return to the 10% level is 30 seconds or less. In addition, the measurement apparatus has a drift-free zero level value, remains stable at substantially the same level, and can thus be used as a highly reliable measurement apparatus. Table 4 contains detailed numeric data related to response characteristics.

**Table 4**

| Response characteristics of embodiment 2, which employs high-conentration hydrogen gas sensor | | | | | |
|---|---|---|---|---|---|
| Hydrogen gas concentration, % | Calorific value, °C | Temperature difference generated, °C | Voltage signal, mV | T90, sec | TD90, sec |
| 0.025 | 0.1 | 0.09 | 0.024 | 36 | 29 |
| 10 | 9.2 | 1.2 | 0.89 | 32 | 24 |
| 20 | 17.8 | 2.4 | 1.87 | 29 | 32 |
| 30 | 28.0 | 4.2 | 3.27 | 33 | 24 |
| 40 | 36.4 | 5.8 | 4.48 | 36 | 24 |
| 50 | 45.5 | 7.5 | 5.82 | 37 | 23 |
| 60 | 58.9 | 10.0 | 7.76 | 40 | 24 |
| 70 | 69.6 | 12.2 | 9.44 | 36 | 23 |
| 80 | 85.3 | 15.2 | 11.69 | 25 | 23 |
| 90 | 100.1 | 18.5 | 13.75 | 35 | 23 |
| 100 | 113.2 | 22.5 | 16.22 | 54 | 22 |

### (4) Evaluation of Temperature Dependence

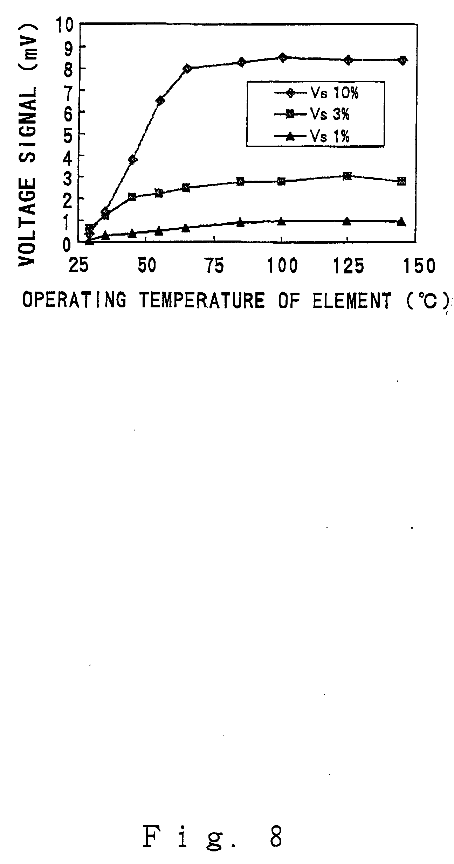

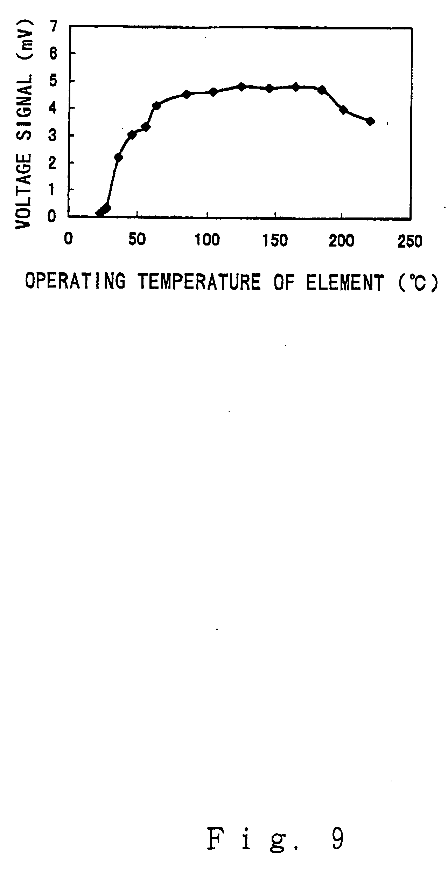

The temperature dependence of the inventive measurement apparatus was evaluated. The results are shown in Fig. 8. It was learned based on the relationship between the voltage signal and the operating temperature of the thermoelectric gas sensor element at various hydrogen concentrations that the thermoelectric hydrogen sensor generates stable output signals at an element temperature of 80°C or greater, has no temperature dependence, and can be used in concentration measurements, as shown in Fig. 5. It was also learned that voltage signal variations can be minimized across a wide range of temperatures (in which the operating temperature of the element ranges from 80 to 180°C) by employing a measurement apparatus comprising the above-described thermoelectric gas sensor to measure the concentration of flammable gas, as shown in Fig. 9.

### INDUSTRIAL APPLICABILITY

As described in detail above, the present invention relates to a gas detection sensor configured such that a detection signal related to flammable gas can be generated by the production of heat due to a catalytic reaction between the flammable gas and a catalyst component. The present invention has the following effects.
(1) It is possible to obtain a flammable gas detection sensor capable of operating at room temperature and providing a response speed measured in units of seconds.
(2) Integration with silicon chips is made possible because the sensor is shaped as a film and can operate at room temperature.
(3) Voltage can be readily generated merely by modifying the print pattern without employing amplifier circuits.
(4) Specific gases can be selectively identified and quantitatively measured in a simple and accurate manner by varying the type of catalyst component on the sensor surface.
(5) It is possible to provide a highly reliable gas detection sensor substantially devoid of the resistance variations normally induced by the detection gas.
(6) It is possible to provide a gas detection sensor having excellent cyclic characteristics.
(7) A high-concentration flammable gas can be measured with high accuracy.
(8) The concentration of flammable gas can be measured within a concentration range of 0.25% to 100%.
(9) The proposed method allows the concentration of hydrogen gas alone in flammable gas to be measured, and stable output signals to be obtained across a wide range of temperatures (80°C to 180°C) by employing, for example, a thermoelectric gas sensor.
(10) It is possible to provide a flammable gas concentration measurement apparatus featuring this measurement method.

## Claims

1. A flammable gas detection sensor for converting heat generated by a catalytic reaction between flammable gas and a catalyst component to a voltage signal by a thermoelectric conversion effect, and detecting the signal as a detection signal, **characterized in** comprising as constituent elements thereof a catalyst component for initiating a catalytic reaction when in contact with a gas to be detected, and a film of thermoelectric conversion material which converts local temperature differences brought about by the heat generated by the reaction to a voltage signal.

2. The gas detection sensor according to claim 1, wherein the catalyst component for initiating a catalytic reaction is at least one selected from platinum (Pt), palladium (Pd), and gold (Au).

3. The gas detection sensor according to claim 1 or 2, **characterized in that** the film of thermoelectric conversion material is selected from BiTe compounds, PbTe compounds. FeSi compounds, SiGe compounds, skutterdite compounds, half-Heusler intermetallic compounds, or metal oxide compounds, which possess enhanced thermoelectric conversion capabilities and minimal gas-induced resistance and thermoelectromotive force variations.

4. The gas detection sensor according to claim 1, **characterized in that** selectivity that provides response to hydrogen gas alone is obtained by the use of a platinum catalyst component.

5. A method for measuring the concentration of flammable gas, **characterized by** comprising, as constituent elements thereof,
(1) sampling part of the flammable gas;
(2) diluting the sample with an air stream at a specific dilution ratio;
(3) measuring the diluted gas using a gas sensor; and
(4) measuring the concentration of the flammable gas on the basis of the measured value.

6. The measurement method according to claim 5, wherein the ratio in which the gas is diluted with an air stream ranges from 1/2 to 1/100.

7. The measurement method according to claim 5, **characterized by** using a flammable gas detection sensor which is a gas detection sensor which converts heat generated by the catalytic reaction between the catalyst component and a flammable gas to a voltage signal by a thermoelectric conversion effect, and detects this signal as a detection signal, and constituent elements of which comprise a catalyst component for initiating a catalytic reaction when in contact with a gas to be detected, and a film of thermoelectric conversion material which converts local temperature differences brought about by heat generated by the reaction to a voltage signal.

8. The measurement method according to claim 7, wherein the concentration of flammable gas is measured within a concentration range of 0.25% to 100%.

9. The measurement method according to claim 7, wherein platinum (Pt) is used for the catalyst component.

10. The measurement method according to claim 7, **characterized in that** the measurements are carried out within a range in which the operating temperature of the gas sensor varies from 60°C to 180°C.

11. A measurement apparatus based on the use of the method according to any of claims 5 through 10, **characterized by** comprising as constituent elements thereof a sampling unit for sampling part of the flammable gas, a mixer unit for diluting the sample with an air stream, and a gas sensor unit for measuring the diluted gas.

12. The measurement apparatus according to claim 11, **characterized by** using a flammable gas detection sensor which is a gas detection sensor which converts the heat generated by the catalytic reaction between the catalyst component and a flammable gas to a voltage signal by a thermoelectric conversion effect, and detects this signal as a detection signal, and constituent elements of which comprise a catalyst component for initiating a catalytic reaction when in contact with a gas to be detected, and a film of thermoelectric conversion material which converts local temperature differences brought about by heat generated by the reaction to a voltage signal.
